# EUROPEAN PATENT APPLICATION

(11) **EP 1 172 444 A1**
(43) Date of publication of application: **16.01.2002**
(21) Application number: 00114786.7
(22) Date of filing: 10.07.2000
(51) Int. Cl.: C12Q 1/68

(54) **Diagnostic use of polymorphisms in the gene coding for the TNF Receptor II and method for detecting non-responders to Anti-TNF therapy**

(71) Applicant: Conaris Research Institute GmbH, 24105 Kiel (DE)
(72) Inventor: SCHREIBER, Stefan, Prof. Dr., 24105 Kiel (DE); HAMPE, Jochen,Dr., 12437 Berlin (DE); MASCHERETTI, Silvia, 24103 Kiel (DE)
(74) Representative: Klunker . Schmitt-Nilson . Hirsch

(57) **Abstract**

The invention relates to a method for detecting non-responders to anti-TNF therapy comprising testing an individual for homozygosity for a single nucleotid polymorphism in the gene coding for the TNF Receptor II. Monoclonal antibodies against TNF-α (infliximab) represent a new treatment for steroid refractory Crohn's disease that result in a remission rate of 30-50% after 4 weeks. Known single nucleotid polymorphisms within the TNF Receptor I and TNF Receptor II were tested for association with the response to the therapy. It was found that individuals homozygote for the mutated allele arginine at amino acid position +196 in the TNF Receptor II or the mutated allele in exon 2 at amino acid position 56 did not respond. Polymorphisms in exon 2 was newly found. None of the individuals homozygote for the mutations in exons 2 or 6 responded. The mutation in exon 2, although a silent mutation, can be used as a marker because it is in a high linkage disequilibrium with the mutation in exon 6.

SEQUENCE LISTING

<110> Conaris Research Institute GmbH

<120> Diagnostic Use of Polymorphisms in the Gene Coding for
      the TNF Receptor II and Method for Detecting
      Non-Responders to Anti-TNF-Therapy

<130> K51347/8

<140>
<141>

<160> 54

<170> PatentIn Ver. 2.1

<210> 1
<211> 21
<212> DNA
<213> Artificial Sequence

<220>
<223> Description of Artificial Sequence: Forward Primer

<400> 1
cttccacgag gtgacatctc c                                           21


<210> 2
<211> 19
<212> DNA
<213> Artificial Sequence

<220>
<223> Description of Artificial Sequence: Reverse Primer

<400> 2
gccctaatac agggccagc                                              19


<210> 3
<211> 21
<212> DNA
<213> Artificial Sequence

<220>
<223> Description of Artificial Sequence: Forward Primer

<400> 3
ggacagattg cagctggaat g                                                21

<210> 4
<211> 20
<212> DNA
<213> Artificial Sequence

<220>
<223> Description of Artificial Sequence: Reverse Primer

<400> 4
tagagccaga ccacctgggt                                                  20

<210> 5
<211> 19
<212> DNA
<213> Artificial Sequence

<220>
<223> Description of Artificial Sequence: Forward Primer

<400> 5
agcctggaca acatggcga                                                   19

<210> 6
<211> 20
<212> DNA
<213> Artificial Sequence

<220>
<223> Description of Artificial Sequence: Reverse Primer

<400> 6
ccctcgactg aaagcgaaag                                                  20

<210> 7
<211> 17
<212> DNA
<213> Artificial Sequence

<220>
<223> Description of Artificial Sequence: Forward Primer

<400> 7
gaggcgtgtc caaggcc                                                    17


<210> 8
<211> 17
<212> DNA
<213> Artificial Sequence

<220>
<223> Description of Artificial Sequence: Reverse Primer

<400> 8
gcgcggagtc accacct                                                    17


<210> 9
<211> 19
<212> DNA
<213> Artificial Sequence

<220>
<223> Description of Artificial Sequence: Forward Primer

<400> 9
atcacccatg gcagaaccc                                                  19


<210> 10
<211> 14
<212> DNA
<213> Artificial Sequence

<220>
<223> Description of Artificial Sequence: Reverse Primer

<400> 10
tgccctcacc cggc                                                       14


<210> 11
<211> 21
<212> DNA
<213> Artificial Sequence

<220>
<223> Description of Artificial Sequence: Forward Primer

<400> 11
gactctggcc ttgtttcctc a                                                21

<210> 12
<211> 18
<212> DNA
<213> Artificial Sequence

<220>
<223> Description of Artificial Sequence: Reverse Primer

<400> 12
gggaagttgg aggcaggg                                                    18

<210> 13
<211> 17
<212> DNA
<213> Artificial Sequence

<220>
<223> Description of Artificial Sequence: Forward Primer

<400> 13
tgaccgtttg cgccctc                                                     17

<210> 14
<211> 19
<212> DNA
<213> Artificial Sequence

<220>
<223> Description of Artificial Sequence: Reverse Primer

<400> 14
gtccccaagg acctgagcc                                                   19

<210> 15
<211> 18
<212> DNA
<213> Artificial Sequence

<220>
<223> Description of Artificial Sequence: Forward Primer

<400> 15
agacagagct ccttgggc                                                    18

<210> 16
<211> 21
<212> DNA
<213> Artificial Sequence

<220>
<223> Description of Artificial Sequence: Reverse Primer

<400> 16
gcagacagaa ggagtgaatg a                                                21

<210> 17
<211> 17
<212> DNA
<213> Artificial Sequence

<220>
<223> Description of Artificial Sequence: Forward Primer

<400> 17
tcctggcttg ctggctg                                                     17

<210> 18
<211> 17
<212> DNA
<213> Artificial Sequence

<220>
<223> Description of Artificial Sequence: Reverse Primer

<400> 18
gagggcagtg gagacac                                                     17

<210> 19
<211> 17
<212> DNA
<213> Artificial Sequence

<220>
<223> Description of Artificial Sequence: Forward Primer

<400> 19
gctgactgct ctcccct                                                    17

<210> 20
<211> 18
<212> DNA
<213> Artificial Sequence

<220>
<223> Description of Artificial Sequence: Reverse Primer

<400> 20
tgggaagaag caggtgtg                                                   18

<210> 21
<211> 20
<212> DNA
<213> Artificial Sequence

<220>
<223> Description of Artificial Sequence: Forward Primer

<400> 21
gaatctgcat cttgggcagg                                                 20

<210> 22
<211> 17
<212> DNA
<213> Artificial Sequence

<220>
<223> Description of Artificial Sequence: Reverse Primer

<400> 22
gaggctgcgg ctgtgga                                                    17

<210> 23
<211> 19
<212> DNA
<213> Artificial Sequence

<220>
<223> Description of Artificial Sequence: Forward Primer

<400> 23
cggtgtgggc tgtgtcgta                                                    19

<210> 24
<211> 19
<212> DNA
<213> Artificial Sequence

<220>
<223> Description of Artificial Sequence: Reverse Primer

<400> 24
cctacagggc tgccacctc                                                    19

<210> 25
<211> 38
<212> DNA
<213> Artificial Sequence

<220>
<223> Description of Artificial Sequence: FAM Probe

<400> 25
acagatccag acaggttcag ttatgtgtct gagaagtt                               38

<210> 26
<211> 38
<212> DNA
<213> Artificial Sequence

<220>
<223> Description of Artificial Sequence: TET Probe

<400> 26
acagatccag acagtttcag ttatgtgtct gagaagtt                               38

<210> 27
<211> 24
<212> DNA
<213> Artificial Sequence

<220>
<223> Description of Artificial Sequence: Forward Primer

<400> 27
gacaggttat ctccactctg caaa                                          24

<210> 28
<211> 26
<212> DNA
<213> Artificial Sequence

<220>
<223> Description of Artificial Sequence: Reverse Primer

<400> 28
caattcagaa tgcttagctt tttagc                                        26

<210> 29
<211> 22
<212> DNA
<213> Artificial Sequence

<220>
<223> Description of Artificial Sequence: FAM Probe

<400> 29
tgctgctgcc gctggtgaga cc                                            22

<210> 30
<211> 25
<212> DNA
<213> Artificial Sequence

<220>
<223> Description of Artificial Sequence: TET Probe

<400> 30
aactgctgct gccactggtg agacc                                         25

<210> 31
<211> 21
<212> DNA
<213> Artificial Sequence

<220>
<223> Description of Artificial Sequence: Forward Primer

<400> 31
cttgggacgt cctggacaga c                                                21

<210> 32
<211> 17
<212> DNA
<213> Artificial Sequence

<220>
<223> Description of Artificial Sequence: Reverse Primer

<400> 32
aaggtgcctc gcccacc                                                     17

<210> 33
<211> 21
<212> DNA
<213> Artificial Sequence

<220>
<223> Description of Artificial Sequence: FAM Probe

<400> 33
tgcagcaaat gctcgccggg t                                                21

<210> 34
<211> 20
<212> DNA
<213> Artificial Sequence

<220>
<223> Description of Artificial Sequence: TET Probe

<400> 34
tgcagcaagt gctcgccggg                                                  20

<210> 35
<211> 27
<212> DNA
<213> Artificial Sequence

<220>
<223> Description of Artificial Sequence: Forward Primer

<400> 35
cagagaatac tatgaccaga cagctca                                          27

<210> 36
<211> 16
<212> DNA
<213> Artificial Sequence

<220>
<223> Description of Artificial Sequence: Reverse Primer

<400> 36
gagtgccccc gtggct                                                      16

<210> 37
<211> 26
<212> DNA
<213> Artificial Sequence

<220>
<223> Description of Artificial Sequence: FAM Probe

<400> 37
aatgcaagca tggatgcagt ctgcac                                           26

<210> 38
<211> 26
<212> DNA
<213> Artificial Sequence

<220>
<223> Description of Artificial Sequence: TET Probe

<400> 38
aatgcaagca gggatgcagt ctgcac                                           26

<210> 39
<211> 20
<212> DNA
<213> Artificial Sequence

<220>
<223> Description of Artificial Sequence: Forward Primer

<400> 39
gctgtaacgt ggtggccatc                                                    20

<210> 40
<211> 15
<212> DNA
<213> Artificial Sequence

<220>
<223> Description of Artificial Sequence: Reverse Primer

<400> 40
ctgggttctg gagtt                                                         15

<210> 41
<211> 26
<212> DNA
<213> Artificial Sequence

<220>
<223> Description of Artificial Sequence: FAM Probe

<400> 41
agaggcagcg agttgtggaa agcctc                                             26

<210> 42
<211> 23
<212> DNA
<213> Artificial Sequence

<220>
<223> Description of Artificial Sequence: TET Probe

<400> 42
aggcagcggg ttgtggaaag cct                                                23

<210> 43
<211> 25
<212> DNA
<213> Artificial Sequence

<220>
<223> Description of Artificial Sequence: Forward Primer

<400> 43
accactagga ctctgaggct ctttc                                          25


<210> 44
<211> 17
<212> DNA
<213> Artificial Sequence

<220>
<223> Description of Artificial Sequence: Reverse Primer

<400> 44
ccagccagcc ttccgag                                                   17


<210> 45
<211> 22
<212> DNA
<213> Artificial Sequence

<220>
<223> Description of Artificial Sequence: FAM Probe

<400> 45
cctctgctgc catggcgtgt cc                                             22


<210> 46
<211> 23
<212> DNA
<213> Artificial Sequence

<220>
<223> Description of Artificial Sequence: TET Probe#

<400> 46
cctctgctgc catggtgtgt cct                                            23


<210> 47
<211> 19
<212> DNA
<213> Artificial Sequence

<220>
<223> Description of Artificial Sequence: Forward Primer

<400> 47
ctgcaggcca agagcagag                                                    19

<210> 48
<211> 21
<212> DNA
<213> Artificial Sequence

<220>
<223> Description of Artificial Sequence: Reverse Primer

<400> 48
ggttttctgg aagccagagc t                                                 21

<210> 49
<211> 3683
<212> DNA
<213> Homo sapiens

<220>
<221> CDS
<222> (90)..(1475)

<220>
<221> mat_peptide
<222> (156)

<400> 49
gcgagcgcag cggagcctgg agagaaggcg ctgggctgcg agggcgcgag ggcgcgaggg 60

caggggggcaa ccggaccccg cccgcaccc atg gcg ccc gtc gcc gtc tgg gcc    113
                                 Met Ala Pro Val Ala Val Trp Ala
                                         -20                 -15

gcg ctg gcc gtc gga ctg gag ctc tgg gct gcg gcg cac gcc ttg ccc    161
Ala Leu Ala Val Gly Leu Glu Leu Trp Ala Ala Ala His Ala Leu Pro
            -10                 -5                  -1   1

gcc cag gtg gca ttt aca ccc tac gcc ccg gag ccc ggg agc aca tgc    209
Ala Gln Val Ala Phe Thr Pro Tyr Ala Pro Glu Pro Gly Ser Thr Cys
        5                   10                  15

cgg ctc aga gaa tac tat gac cag aca gct cag atg tgc tgc agc aaa    257
Arg Leu Arg Glu Tyr Tyr Asp Gln Thr Ala Gln Met Cys Cys Ser Lys
    20                  25                  30

tgc tcg ccg ggc caa cat gca aaa gtc ttc tgt acc aag acc tcg gac    305
Cys Ser Pro Gly Gln His Ala Lys Val Phe Cys Thr Lys Thr Ser Asp
 35                  40                  45                  50

acc gtg tgt gac tcc tgt gag gac agc aca tac acc cag ctc tgg aac    353
Thr Val Cys Asp Ser Cys Glu Asp Ser Thr Tyr Thr Gln Leu Trp Asn
                 55                  60                  65

tgg gtt ccc gag tgc ttg agc tgt ggc tcc cgc tgt agc tct gac cag    401
Trp Val Pro Glu Cys Leu Ser Cys Gly Ser Arg Cys Ser Ser Asp Gln
             70                  75                  80

gtg gaa act caa gcc tgc act cgg gaa cag aac cgc atc tgc acc tgc    449
Val Glu Thr Gln Ala Cys Thr Arg Glu Gln Asn Arg Ile Cys Thr Cys
         85                  90                  95

agg ccc ggc tgg tac tgc gcg ctg agc aag cag gag ggg tgc cgg ctg    497
Arg Pro Gly Trp Tyr Cys Ala Leu Ser Lys Gln Glu Gly Cys Arg Leu
    100                 105                 110

tgc gcg ccg ctg cgc aag tgc cgc ccg ggc ttc ggc gtg gcc aga cca    545
Cys Ala Pro Leu Arg Lys Cys Arg Pro Gly Phe Gly Val Ala Arg Pro
115                 120                 125                 130

gga act gaa aca tca gac gtg gtg tgc aag ccc tgt gcc ccg ggg acg    593
Gly Thr Glu Thr Ser Asp Val Val Cys Lys Pro Cys Ala Pro Gly Thr
                135                 140                 145

ttc tcc aac acg act tca tcc acg gat att tgc agg ccc cac cag atc    641
Phe Ser Asn Thr Thr Ser Ser Thr Asp Ile Cys Arg Pro His Gln Ile
            150                 155                 160

tgt aac gtg gtg gcc atc cct ggg aat gca agc atg gat gca gtc tgc    689
Cys Asn Val Val Ala Ile Pro Gly Asn Ala Ser Met Asp Ala Val Cys
        165                 170                 175

acg tcc acg tcc ccc acc cgg agt atg gcc cca ggg gca gta cac tta    737
Thr Ser Thr Ser Pro Thr Arg Ser Met Ala Pro Gly Ala Val His Leu
    180                 185                 190

ccc cag cca gtg tcc aca cga tcc caa cac acg cag cca act cca gaa    785
Pro Gln Pro Val Ser Thr Arg Ser Gln His Thr Gln Pro Thr Pro Glu
195                 200                 205                 210

ccc agc act gct cca agc acc tcc ttc ctg ctc cca atg ggc ccc agc    833
Pro Ser Thr Ala Pro Ser Thr Ser Phe Leu Leu Pro Met Gly Pro Ser
                215                 220                 225

ccc cca gct gaa ggg agc act ggc gac ttc gct ctt cca gtt gga ctg 881
Pro Pro Ala Glu Gly Ser Thr Gly Asp Phe Ala Leu Pro Val Gly Leu
230 235 240

att gtg ggt gtg aca gcc ttg ggt cta cta ata ata gga gtg gtg aac 929
Ile Val Gly Val Thr Ala Leu Gly Leu Leu Ile Ile Gly Val Val Asn
                245                 250                 255

tgt gtc atc atg acc cag gtg aaa aag aag ccc ttg tgc ctg cag aga 977
Cys Val Ile Met Thr Gln Val Lys Lys Lys Pro Leu Cys Leu Gln Arg
            260                 265                 270

gaa gcc aag gtg cct cac ttg cct gcc gat aag gcc cgg ggt aca cag 1025
Glu Ala Lys Val Pro His Leu Pro Ala Asp Lys Ala Arg Gly Thr Gln
275 280 285 290

ggc ccc gag cag cag cac ctg ctg atc aca gcg ccg agc tcc agc agc 1073
Gly Pro Glu Gln Gln His Leu Leu Ile Thr Ala Pro Ser Ser Ser Ser
295 300 305

agc tcc ctg gag agc tcg gcc agt gcg ttg gac aga agg gcg ccc act 1121
Ser Ser Leu Glu Ser Ser Ala Ser Ala Leu Asp Arg Arg Ala Pro Thr
310 315 320

cgg aac cag cca cag gca cca ggc gtg gag gcc agt ggg gcc ggg gag 1169
Arg Asn Gln Pro Gln Ala Pro Gly Val Glu Ala Ser Gly Ala Gly Glu
                325                 330                 335

gcc cgg gcc agc acc ggg agc tca gat tct tcc cct ggt ggc cat ggg 1217
Ala Arg Ala Ser Thr Gly Ser Ser Asp Ser Ser Pro Gly Gly His Gly
            340                 345                 350

acc cag gtc aat gtc acc tgc atc gtg aac gtc tgt agc agc tct gac 1265
Thr Gln Val Asn Val Thr Cys Ile Val Asn Val Cys Ser Ser Ser Asp
355 360 365 370

cac agc tca cag tgc tcc tcc caa gcc agc tcc aca atg gga gac aca 1313
His Ser Ser Gln Cys Ser Ser Gln Ala Ser Ser Thr Met Gly Asp Thr
375 380 385

gat tcc agc ccc tcg gag tcc ccg aag gac gag cag gtc ccc ttc tcc 1361
Asp Ser Ser Pro Ser Glu Ser Pro Lys Asp Glu Gln Val Pro Phe Ser
390 395 400

aag gag gaa tgt gcc ttt cgg tca cag ctg gag acg cca gag acc ctg 1409
Lys Glu Glu Cys Ala Phe Arg Ser Gln Leu Glu Thr Pro Glu Thr Leu
                405                 410                 415

ctg ggg agc acc gaa gag aag ccc ctg ccc ctt gga gtg cct gat gct 1457
Leu Gly Ser Thr Glu Glu Lys Pro Leu Pro Leu Gly Val Pro Asp Ala
            420                 425                 430

ggg atg aag ccc agt taa ccaggccggt gtgggctgtg tcgtagccaa 1505
Gly Met Lys Pro Ser
435 440

ggtgggctga gccctggcag gatgaccctg cgaaggggcc ctggtccttc caggccccca 1565

ccactaggac tctgaggctc tttctgggcc aagttcctct agtgccctcc acagccgcag 1625

cctccctctg acctgcaggc caagagcaga ggcagcgagt tggggaaagc ctctgctgcc 1685

atggtgtgtc cctctcggaa ggctggctgg gcatggacgt tcggggcatg ctggggcaag 1745

tccctgactc tctgtgacct gccccgccca gctgcacctg ccagcctggc ttctggagcc 1805

cttgggtttt ttgtttgttt gtttgtttgt ttgtttgttt ctccccctgg gctctgccca 1865

gctctggctt ccagaaaacc ccagcatcct tttctgcaga ggggctttct ggagaggagg 1925

gatgctgcct gagtcaccca tgaagacagg acagtgcttc agcctgaggc tgagactgcg 1985

ggatggtcct ggggctctgt gtagggagga ggtggcagcc ctgtagggaa cggggtcctt 2045

caagttagct caggaggctt ggaaagcatc acctcaggcc aggtgcagtg gctcacgcct 2105

atgatcccag cactttggga ggctgaggcg ggtggatcac ctgaggttag gagttcgaga 2165

ccagcctggc caacatggta aaaccccatc tctactaaaa atacagaaat tagccgggcg 2225

tggtggcggg cacctatagt cccagctact cagaagcctg aggctgggaa atcgtttgaa 2285

cccgggaagc ggaggttgca gggagccgag atcacgccac tgcactccag cctgggcgac 2345

agagcgagag tctgtctcaa aagaaaaaaa aaaaagcacc gcctccaaat gctaacttgt 2405

ccttttgtac catggtgtga aagtcagatg cccagagggc ccaggcaggc caccatattc 2465

agtgctgtgg cctgggcaag ataacgcact tctaactaga aatctgccaa ttttttaaaa 2525

aagtaagtac cactcaggcc aacaagccaa cgacaaagcc aaactctgcc agccacatcc 2585

aaccccccac ctgccatttg caccctccgc cttcactccg gtgtgcctgc agccccgcgc 2645

ctccttcctt gctgtcctag gccacaccat ctcctttcag ggaatttcag gaactagaga 2705

tgactgagtc ctcgtagcca tctctctact cctacctcag cctagaccct cctcctcccc 2765

cagaggggtg ggttcctctt ccccactccc caccttcaat tcctgggccc caaacgggct 2825

gccctgccac tttggtacat ggccagtgtg atcccaagtg ccagtcttgt gtctgcgtct 2885

gtgttgcgtg tcgtgggtgt gtgtagccaa ggtcggtaag ttgaatggcc tgccttgaag 2945

ccactgaagc tgggattcct ccccattaga gtcagccttc cccctcccag ggccagggcc 3005

ctgcagaggg gaaaccagtg tagccttgcc cggattctgg gaggaagcag gttgaggggc 3065

tcctggaaag gctcagtctc aggagcatgg ggataaagga gaaggcatga aattgtctag 3125

cagagcaggg gcagggtgat aaattgttga taaattccac tggacttgag cttggcagct 3185

gaactattgg agggtgggag agcccagcca ttaccatgga gacaagaagg gttttccacc 3245

ctggaatcaa gatgtcagac tggctggctg cagtgacgtg cacctgtact caggaggctg 3305

aggggaggat cactggagcc caggagtttg aggctgcagc gagctatgat cgcgccacta 3365

cactccagcc tgagcaacag agtgagaccc tgtctcttaa agaaaaaaaa agtcagactg 3425

ctgggactgg ccaggtttct gcccacattg gacccacatg aggacatgat ggagcgcacc 3485

tgccccctgg tggacagtcc tgggagaacc tcaggcttcc ttggcatcac agggcagagc 3545

cgggaagcga tgaatttgga gactctgtgg ggccttggtt cccttgtgtg tgtgtgttga 3605

tcccaagaca atgaaagttt gcactgtatg ctggacggca ttcctgctta tcaataaacc 3665

tgtttgtttt aaaaaaaa 3683

<210> 50
<211> 461
<212> PRT
<213> Homo sapiens

<400> 50
Met Ala Pro Val Ala Val Trp Ala Ala Leu Ala Val Gly Leu Glu Leu
1               5                   10                  15
Trp Ala Ala Ala His Ala Leu Pro Ala Gln Val Ala Phe Thr Pro Tyr
            20                  25                  30
Ala Pro Glu Pro Gly Ser Thr Cys Arg Leu Arg Glu Tyr Tyr Asp Gln
        35                  40                  45

Thr Ala Gln Met Cys Cys Ser Lys Cys Ser Pro Gly Gln His Ala Lys
50                  55                  60
Val Phe Cys Thr Lys Thr Ser Asp Thr Val Cys Asp Ser Cys Glu Asp
65                  70                  75                  80
Ser Thr Tyr Thr Gln Leu Trp Asn Trp Val Pro Glu Cys Leu Ser Cys
                85                  90                  95
Gly Ser Arg Cys Ser Ser Asp Gln Val Glu Thr Gln Ala Cys Thr Arg
            100                 105                 110
Glu Gln Asn Arg Ile Cys Thr Cys Arg Pro Gly Trp Tyr Cys Ala Leu
        115                 120                 125
Ser Lys Gln Glu Gly Cys Arg Leu Cys Ala Pro Leu Arg Lys Cys Arg
    130                 135                 140
Pro Gly Phe Gly Val Ala Arg Pro Gly Thr Glu Thr Ser Asp Val Val
145                 150                 155                 160
Cys Lys Pro Cys Ala Pro Gly Thr Phe Ser Asn Thr Thr Ser Ser Thr
                165                 170                 175
Asp Ile Cys Arg Pro His Gln Ile Cys Asn Val Val Ala Ile Pro Gly
            180                 185                 190
Asn Ala Ser Met Asp Ala Val Cys Thr Ser Thr Ser Pro Thr Arg Ser
        195                 200                 205
Met Ala Pro Gly Ala Val His Leu Pro Gln Pro Val Ser Thr Arg Ser
    210                 215                 220
Gln His Thr Gln Pro Thr Pro Glu Pro Ser Thr Ala Pro Ser Thr Ser
225                 230                 235                 240
Phe Leu Leu Pro Met Gly Pro Ser Pro Pro Ala Glu Gly Ser Thr Gly
                245                 250                 255
Asp Phe Ala Leu Pro Val Gly Leu Ile Val Gly Val Thr Ala Leu Gly
            260                 265                 270
Leu Leu Ile Ile Gly Val Val Asn Cys Val Ile Met Thr Gln Val Lys
        275                 280                 285
Lys Lys Pro Leu Cys Leu Gln Arg Glu Ala Lys Val Pro His Leu Pro
    290                 295                 300
Ala Asp Lys Ala Arg Gly Thr Gln Gly Pro Glu Gln Gln His Leu Leu
305                 310                 315                 320
Ile Thr Ala Pro Ser Ser Ser Ser Ser Ser Leu Glu Ser Ser Ala Ser
                325                 330                 335
Ala Leu Asp Arg Arg Ala Pro Thr Arg Asn Gln Pro Gln Ala Pro Gly
            340                 345                 350
Val Glu Ala Ser Gly Ala Gly Glu Ala Arg Ala Ser Thr Gly Ser Ser
        355                 360                 365
Asp Ser Ser Pro Gly Gly His Gly Thr Gln Val Asn Val Thr Cys Ile
    370                 375                 380
Val Asn Val Cys Ser Ser Ser Asp His Ser Ser Gln Cys Ser Ser Gln
385                 390                 395                 400
Ala Ser Ser Thr Met Gly Asp Thr Asp Ser Ser Pro Ser Glu Ser Pro
                405                 410                 415
Lys Asp Glu Gln Val Pro Phe Ser Lys Glu Glu Cys Ala Phe Arg Ser
            420                 425                 430

Gln Leu Glu Thr Pro Glu Thr Leu Leu Gly Ser Thr Glu Glu Lys Pro
        435                 440                 445
Leu Pro Leu Gly Val Pro Asp Ala Gly Met Lys Pro Ser
    450                 455                 460

<210> 51
<211> 3683
<212> DNA
<213> Homo sapiens

<220>
<221> CDS
<222> (90)..(1475)

<220>
<221> mat_peptide
<222> (156)

<400> 51
gcgagcgcag cggagcctgg agagaaggcg ctgggctgcg agggcgcgag ggcgcgaggg 60

caggggggcaa ccggaccccg cccgcaccc atg gcg ccc gtc gcc gtc tgg gcc 113
                                 Met Ala Pro Val Ala Val Trp Ala
                                         -20                 -15

gcg ctg gcc gtc gga ctg gag ctc tgg gct gcg gcg cac gcc ttg ccc 161
Ala Leu Ala Val Gly Leu Glu Leu Trp Ala Ala Ala His Ala Leu Pro
                -10                 -5                  -1   1

gcc cag gtg gca ttt aca ccc tac gcc ccg gag ccc ggg agc aca tgc 209
Ala Gln Val Ala Phe Thr Pro Tyr Ala Pro Glu Pro Gly Ser Thr Cys
        5                   10                  15

cgg ctc aga gaa tac tat gac cag aca gct cag atg tgc tgc agc aag 257
Arg Leu Arg Glu Tyr Tyr Asp Gln Thr Ala Gln Met Cys Cys Ser Lys
    20                  25                  30

tgc tcg ccg ggc caa cat gca aaa gtc ttc tgt acc aag acc tcg gac 305
Cys Ser Pro Gly Gln His Ala Lys Val Phe Cys Thr Lys Thr Ser Asp
35                  40                  45                  50

acc gtg tgt gac tcc tgt gag gac agc aca tac acc cag ctc tgg aac 353
Thr Val Cys Asp Ser Cys Glu Asp Ser Thr Tyr Thr Gln Leu Trp Asn
                55                  60                  65

tgg gtt ccc gag tgc ttg agc tgt ggc tcc cgc tgt agc tct gac cag 401

Trp Val Pro Glu Cys Leu Ser Cys Gly Ser Arg Cys Ser Ser Asp Gln
            70                  75                  80

gtg gaa act caa gcc tgc act cgg gaa cag aac cgc atc tgc acc tgc     449
Val Glu Thr Gln Ala Cys Thr Arg Glu Gln Asn Arg Ile Cys Thr Cys
        85                  90                  95

agg ccc ggc tgg tac tgc gcg ctg agc aag cag gag ggg tgc cgg ctg     497
Arg Pro Gly Trp Tyr Cys Ala Leu Ser Lys Gln Glu Gly Cys Arg Leu
    100                 105                 110

tgc gcg ccg ctg cgc aag tgc cgc ccg ggc ttc ggc gtg gcc aga cca     545
Cys Ala Pro Leu Arg Lys Cys Arg Pro Gly Phe Gly Val Ala Arg Pro
115                 120                 125                 130

gga act gaa aca tca gac gtg gtg tgc aag ccc tgt gcc ccg ggg acg     593
Gly Thr Glu Thr Ser Asp Val Val Cys Lys Pro Cys Ala Pro Gly Thr
                135                 140                 145

ttc tcc aac acg act tca tcc acg gat att tgc agg ccc cac cag atc     641
Phe Ser Asn Thr Thr Ser Ser Thr Asp Ile Cys Arg Pro His Gln Ile
            150                 155                 160

tgt aac gtg gtg gcc atc cct ggg aat gca agc atg gat gca gtc tgc     689
Cys Asn Val Val Ala Ile Pro Gly Asn Ala Ser Met Asp Ala Val Cys
        165                 170                 175

acg tcc acg tcc ccc acc cgg agt atg gcc cca ggg gca gta cac tta     737
Thr Ser Thr Ser Pro Thr Arg Ser Met Ala Pro Gly Ala Val His Leu
    180                 185                 190

ccc cag cca gtg tcc aca cga tcc caa cac acg cag cca act cca gaa     785
Pro Gln Pro Val Ser Thr Arg Ser Gln His Thr Gln Pro Thr Pro Glu
195                 200                 205                 210

ccc agc act gct cca agc acc tcc ttc ctg ctc cca atg ggc ccc agc     833
Pro Ser Thr Ala Pro Ser Thr Ser Phe Leu Leu Pro Met Gly Pro Ser
                215                 220                 225

ccc cca gct gaa ggg agc act ggc gac ttc gct ctt cca gtt gga ctg     881
Pro Pro Ala Glu Gly Ser Thr Gly Asp Phe Ala Leu Pro Val Gly Leu
            230                 235                 240

att gtg ggt gtg aca gcc ttg ggt cta cta ata ata gga gtg gtg aac     929
Ile Val Gly Val Thr Ala Leu Gly Leu Leu Ile Ile Gly Val Val Asn
        245                 250                 255

tgt gtc atc atg acc cag gtg aaa aag aag ccc ttg tgc ctg cag aga     977

Cys Val Ile Met Thr Gln Val Lys Lys Lys Pro Leu Cys Leu Gln Arg
            260                 265                 270

gaa gcc aag gtg cct cac ttg cct gcc gat aag gcc cgg ggt aca cag 1025
Glu Ala Lys Val Pro His Leu Pro Ala Asp Lys Ala Arg Gly Thr Gln
275 280 285 290

ggc ccc gag cag cag cac ctg ctg atc aca gcg ccg agc tcc agc agc 1073
Gly Pro Glu Gln Gln His Leu Leu Ile Thr Ala Pro Ser Ser Ser Ser
295 300 305

agc tcc ctg gag agc tcg gcc agt gcg ttg gac aga agg gcg ccc act 1121
Ser Ser Leu Glu Ser Ser Ala Ser Ala Leu Asp Arg Arg Ala Pro Thr
310 315 320

cgg aac cag cca cag gca cca ggc gtg gag gcc agt ggg gcc ggg gag 1169
Arg Asn Gln Pro Gln Ala Pro Gly Val Glu Ala Ser Gly Ala Gly Glu
                325                 330                 335

gcc cgg gcc agc acc ggg agc tca gat tct tcc cct ggt ggc cat ggg 1217
Ala Arg Ala Ser Thr Gly Ser Ser Asp Ser Ser Pro Gly Gly His Gly
            340                 345                 350

acc cag gtc aat gtc acc tgc atc gtg aac gtc tgt agc agc tct gac 1265
Thr Gln Val Asn Val Thr Cys Ile Val Asn Val Cys Ser Ser Ser Asp
355 360 365 370

cac agc tca cag tgc tcc tcc caa gcc agc tcc aca atg gga gac aca 1313
His Ser Ser Gln Cys Ser Ser Gln Ala Ser Ser Thr Met Gly Asp Thr
375 380 385

gat tcc agc ccc tcg gag tcc ccg aag gac gag cag gtc ccc ttc tcc 1361
Asp Ser Ser Pro Ser Glu Ser Pro Lys Asp Glu Gln Val Pro Phe Ser
390 395 400

aag gag gaa tgt gcc ttt cgg tca cag ctg gag acg cca gag acc ctg 1409
Lys Glu Glu Cys Ala Phe Arg Ser Gln Leu Glu Thr Pro Glu Thr Leu
                405                 410                 415

ctg ggg agc acc gaa gag aag ccc ctg ccc ctt gga gtg cct gat gct 1457
Leu Gly Ser Thr Glu Glu Lys Pro Leu Pro Leu Gly Val Pro Asp Ala
            420                 425                 430

ggg atg aag ccc agt taa ccaggccggt gtgggctgtg tcgtagccaa 1505
Gly Met Lys Pro Ser
435 440

ggtgggctga gccctggcag gatgaccctg cgaaggggcc ctggtccttc caggccccca 1565

ccactaggac tctgaggctc tttctgggcc aagttcctct agtgccctcc acagccgcag 1625

cctccctctg acctgcaggc caagagcaga ggcagcgagt tggggaaagc ctctgctgcc 1685

atggtgtgtc cctctcggaa ggctggctgg gcatggacgt tcggggcatg ctggggcaag 1745

tccctgactc tctgtgacct gccccgccca gctgcacctg ccagcctggc ttctggagcc 1805

cttgggtttt ttgtttgttt gtttgtttgt ttgtttgttt ctccccctgg gctctgccca 1865

gctctggctt ccagaaaacc ccagcatcct tttctgcaga ggggctttct ggagaggagg 1925

gatgctgcct gagtcaccca tgaagacagg acagtgcttc agcctgaggc tgagactgcg 1985

ggatggtcct ggggctctgt gtagggagga ggtggcagcc ctgtagggaa cggggtcctt 2045

caagttagct caggaggctt ggaaagcatc acctcaggcc aggtgcagtg gctcacgcct 2105

atgatcccag cactttggga ggctgaggcg ggtggatcac ctgaggttag gagttcgaga 2165

ccagcctggc caacatggta aaaccccatc tctactaaaa atacagaaat tagccgggcg 2225

tggtggcggg cacctatagt cccagctact cagaagcctg aggctgggaa atcgtttgaa 2285

cccgggaagc ggaggttgca gggagccgag atcacgccac tgcactccag cctgggcgac 2345

agagcgagag tctgtctcaa aagaaaaaaa aaaaagcacc gcctccaaat gctaacttgt 2405

ccttttgtac catggtgtga aagtcagatg cccagagggc ccaggcaggc caccatattc 2465

agtgctgtgg cctgggcaag ataacgcact tctaactaga aatctgccaa ttttttaaaa 2525

aagtaagtac cactcaggcc aacaagccaa cgacaaagcc aaactctgcc agccacatcc 2585

aaccccccac ctgccatttg caccctccgc cttcactccg gtgtgcctgc agccccgcgc 2645

ctccttcctt gctgtcctag gccacaccat ctcctttcag ggaatttcag gaactagaga 2705

tgactgagtc ctcgtagcca tctctctact cctacctcag cctagaccct cctcctcccc 2765

cagaggggtg ggttcctctt ccccactccc caccttcaat tcctgggccc caaacgggct 2825

gccctgccac tttggtacat ggccagtgtg atcccaagtg ccagtcttgt gtctgcgtct 2885

gtgttgcgtg tcgtgggtgt gtgtagccaa ggtcggtaag ttgaatggcc tgccttgaag 2945

ccactgaagc tgggattcct ccccattaga gtcagccttc cccctcccag ggccagggcc 3005

ctgcagaggg gaaaccagtg tagccttgcc cggattctgg gaggaagcag gttgaggggc 3065

tcctggaaag gctcagtctc aggagcatgg ggataaagga gaaggcatga aattgtctag 3125

cagagcaggg gcagggtgat aaattgttga taaattccac tggacttgag cttggcagct 3185

gaactattgg agggtgggag agcccagcca ttaccatgga gacaagaagg gttttccacc 3245

ctggaatcaa gatgtcagac tggctggctg cagtgacgtg cacctgtact caggaggctg 3305

aggggaggat cactggagcc caggagtttg aggctgcagc gagctatgat cgcgccacta 3365

cactccagcc tgagcaacag agtgagaccc tgtctcttaa agaaaaaaaa agtcagactg 3425

ctgggactgg ccaggtttct gcccacattg gacccacatg aggacatgat ggagcgcacc 3485

tgccccctgg tggacagtcc tgggagaacc tcaggcttcc ttggcatcac agggcagagc 3545

cgggaagcga tgaatttgga gactctgtgg ggccttggtt cccttgtgtg tgtgtgttga 3605

tcccaagaca atgaaagttt gcactgtatg ctggacggca ttcctgctta tcaataaacc 3665

tgtttgtttt aaaaaaaa 3683

<210> 52
<211> 461
<212> PRT
<213> Homo sapiens

<400> 52
Met Ala Pro Val Ala Val Trp Ala Ala Leu Ala Val Gly Leu Glu Leu
1               5                   10                  15
Trp Ala Ala Ala His Ala Leu Pro Ala Gln Val Ala Phe Thr Pro Tyr
            20                  25                  30
Ala Pro Glu Pro Gly Ser Thr Cys Arg Leu Arg Glu Tyr Tyr Asp Gln
        35                  40                  45
Thr Ala Gln Met Cys Cys Ser Lys Cys Ser Pro Gly Gln His Ala Lys
    50                  55                  60
Val Phe Cys Thr Lys Thr Ser Asp Thr Val Cys Asp Ser Cys Glu Asp
65                  70                  75                  80
Ser Thr Tyr Thr Gln Leu Trp Asn Trp Val Pro Glu Cys Leu Ser Cys
                85                  90                  95
Gly Ser Arg Cys Ser Ser Asp Gln Val Glu Thr Gln Ala Cys Thr Arg
            100                 105                 110
Glu Gln Asn Arg Ile Cys Thr Cys Arg Pro Gly Trp Tyr Cys Ala Leu
        115                 120                 125

Ser Lys Gln Glu Gly Cys Arg Leu Cys Ala Pro Leu Arg Lys Cys Arg
    130                 135                 140
Pro Gly Phe Gly Val Ala Arg Pro Gly Thr Glu Thr Ser Asp Val Val
145                 150                 155                 160
Cys Lys Pro Cys Ala Pro Gly Thr Phe Ser Asn Thr Thr Ser Ser Thr
                165                 170                 175
Asp Ile Cys Arg Pro His Gln Ile Cys Asn Val Val Ala Ile Pro Gly
            180                 185                 190
Asn Ala Ser Met Asp Ala Val Cys Thr Ser Thr Ser Pro Thr Arg Ser
        195                 200                 205
Met Ala Pro Gly Ala Val His Leu Pro Gln Pro Val Ser Thr Arg Ser
    210                 215                 220
Gln His Thr Gln Pro Thr Pro Glu Pro Ser Thr Ala Pro Ser Thr Ser
225                 230                 235                 240
Phe Leu Leu Pro Met Gly Pro Ser Pro Pro Ala Glu Gly Ser Thr Gly
                245                 250                 255
Asp Phe Ala Leu Pro Val Gly Leu Ile Val Gly Val Thr Ala Leu Gly
            260                 265                 270
Leu Leu Ile Ile Gly Val Val Asn Cys Val Ile Met Thr Gln Val Lys
        275                 280                 285
Lys Lys Pro Leu Cys Leu Gln Arg Glu Ala Lys Val Pro His Leu Pro
    290                 295                 300
Ala Asp Lys Ala Arg Gly Thr Gln Gly Pro Glu Gln Gln His Leu Leu
305                 310                 315                 320
Ile Thr Ala Pro Ser Ser Ser Ser Ser Ser Leu Glu Ser Ser Ala Ser
                325                 330                 335
Ala Leu Asp Arg Arg Ala Pro Thr Arg Asn Gln Pro Gln Ala Pro Gly
            340                 345                 350
Val Glu Ala Ser Gly Ala Gly Glu Ala Arg Ala Ser Thr Gly Ser Ser
        355                 360                 365
Asp Ser Ser Pro Gly Gly His Gly Thr Gln Val Asn Val Thr Cys Ile
    370                 375                 380
Val Asn Val Cys Ser Ser Ser Asp His Ser Ser Gln Cys Ser Ser Gln
385                 390                 395                 400
Ala Ser Ser Thr Met Gly Asp Thr Asp Ser Ser Pro Ser Glu Ser Pro
                405                 410                 415
Lys Asp Glu Gln Val Pro Phe Ser Lys Glu Glu Cys Ala Phe Arg Ser
            420                 425                 430
Gln Leu Glu Thr Pro Glu Thr Leu Leu Gly Ser Thr Glu Glu Lys Pro
        435                 440                 445
Leu Pro Leu Gly Val Pro Asp Ala Gly Met Lys Pro Ser
    450                 455                 460

<210> 53
<211> 3683
<212> DNA

<213> Homo sapiens

<220>
<221> CDS
<222> (90)..(1475)

<220>
<221> mat_peptide
<222> (156)

<400> 53
gcgagcgcag cggagcctgg agagaaggcg ctgggctgcg agggcgcgag ggcgcgaggg 60

caggggcaa ccggaccccg cccgcaccc atg gcg ccc gtc gcc gtc tgg gcc 113

-20 -15

gcg ctg gcc gtc gga ctg gag ctc tgg gct gcg gcg cac gcc ttg ccc 161

-10 -5 -1 1

gcc cag gtg gca ttt aca ccc tac gcc ccg gag ccc ggg agc aca tgc 209

                5                   10                  15

cgg ctc aga gaa tac tat gac cag aca gct cag atg tgc tgc agc aag 257

            20                  25                  30

tgc tcg ccg ggc caa cat gca aaa gtc ttc tgt acc aag acc tcg gac 305

35 40 45 50

acc gtg tgt gac tcc tgt gag gac agc aca tac acc cag ctc tgg aac 353

55 60 65

tgg gtt ccc gag tgc ttg agc tgt ggc tcc cgc tgt agc tct gac cag 401

70 75 80

gtg gaa act caa gcc tgc act cgg gaa cag aac cgc atc tgc acc tgc 449

                85                  90                  95

agg ccc ggc tgg tac tgc gcg ctg agc aag cag gag ggg tgc cgg ctg 497

            100                 105                 110

tgc gcg ccg ctg cgc aag tgc cgc ccg ggc ttc ggc gtg gcc aga cca 545

115 120 125 130

gga act gaa aca tca gac gtg gtg tgc aag ccc tgt gcc ccg ggg acg 593

135 140 145

ttc tcc aac acg act tca tcc acg gat att tgc agg ccc cac cag atc 641

150 155 160

tgt aac gtg gtg gcc atc cct ggg aat gca agc agg gat gca gtc tgc 689

                165                 170                 175

acg tcc acg tcc ccc acc cgg agt atg gcc cca ggg gca gta cac tta 737

            180                 185                 190

ccc cag cca gtg tcc aca cga tcc caa cac acg cag cca act cca gaa 785

195 200 205 210

ccc agc act gct cca agc acc tcc ttc ctg ctc cca atg ggc ccc agc 833

215 220 225

ccc cca gct gaa ggg agc act ggc gac ttc gct ctt cca gtt gga ctg 881

230 235 240

att gtg ggt gtg aca gcc ttg ggt cta cta ata ata gga gtg gtg aac 929

                245                 250                 255

tgt gtc atc atg acc cag gtg aaa aag aag ccc ttg tgc ctg cag aga 977

            260                 265                 270

gaa gcc aag gtg cct cac ttg cct gcc gat aag gcc cgg ggt aca cag 1025

275 280 285 290

ggc ccc gag cag cag cac ctg ctg atc aca gcg ccg agc tcc agc agc 1073

295 300 305

agc tcc ctg gag agc tcg gcc agt gcg ttg gac aga agg gcg ccc act 1121

310 315 320

cgg aac cag cca cag gca cca ggc gtg gag gcc agt ggg gcc ggg gag 1169

                325                 330                 335

gcc cgg gcc agc acc ggg agc tca gat tct tcc cct ggt ggc cat ggg 1217

            340                 345                 350

acc cag gtc aat gtc acc tgc atc gtg aac gtc tgt agc agc tct gac 1265

355 360 365 370

cac agc tca cag tgc tcc tcc caa gcc agc tcc aca atg gga gac aca 1313

375 380 385

gat tcc agc ccc tcg gag tcc ccg aag gac gag cag gtc ccc ttc tcc 1361

390 395 400

aag gag gaa tgt gcc ttt cgg tca cag ctg gag acg cca gag acc ctg 1409

                405                 410                 415

ctg ggg agc acc gaa gag aag ccc ctg ccc ctt gga gtg cct gat gct 1457

            420                 425                 430

ggg atg aag ccc agt taa ccaggccggt gtgggctgtg tcgtagccaa 1505

435 440

ggtgggctga gccctggcag gatgaccctg cgaaggggcc ctggtccttc caggccccca 1565

ccactaggac tctgaggctc tttctgggcc aagttcctct agtgccctcc acagccgcag 1625

cctccctctg acctgcaggc caagagcaga ggcagcgagt tggggaaagc ctctgctgcc 1685

atggtgtgtc cctctcggaa ggctggctgg gcatggacgt tcggggcatg ctggggcaag 1745

tccctgactc tctgtgacct gccccgccca gctgcacctg ccagcctggc ttctggagcc 1805

cttgggtttt ttgtttgttt gtttgtttgt ttgtttgttt ctccccctgg gctctgccca 1865

gctctggctt ccagaaaacc ccagcatcct tttctgcaga ggggctttct ggagaggagg 1925

gatgctgcct gagtcaccca tgaagacagg acagtgcttc agcctgaggc tgagactgcg 1985

ggatggtcct ggggctctgt gtagggagga ggtggcagcc ctgtagggaa cggggtcctt 2045

caagttagct caggaggctt ggaaagcatc acctcaggcc aggtgcagtg gctcacgcct 2105

atgatcccag cactttggga ggctgaggcg ggtggatcac ctgaggttag gagttcgaga 2165

ccagcctggc caacatggta aaaccccatc tctactaaaa atacagaaat tagccgggcg 2225

tggtggcggg cacctatagt cccagctact cagaagcctg aggctgggaa atcgtttgaa 2285

cccgggaagc ggaggttgca gggagccgag atcacgccac tgcactccag cctgggcgac 2345

agagcgagag tctgtctcaa aagaaaaaaa aaaaagcacc gcctccaaat gctaacttgt 2405

ccttttgtac catggtgtga aagtcagatg cccagagggc ccaggcaggc caccatattc 2465

agtgctgtgg cctgggcaag ataacgcact tctaactaga aatctgccaa ttttttaaaa 2525

aagtaagtac cactcaggcc aacaagccaa cgacaaagcc aaactctgcc agccacatcc 2585

aaccccccac ctgccatttg caccctccgc cttcactccg gtgtgcctgc agccccgcgc 2645

ctccttcctt gctgtcctag gccacaccat ctcctttcag ggaatttcag gaactagaga 2705

tgactgagtc ctcgtagcca tctctctact cctacctcag cctagaccct cctcctcccc 2765

cagaggggtg ggttcctctt ccccactccc caccttcaat tcctgggccc caaacgggct 2825

gccctgccac tttggtacat ggccagtgtg atcccaagtg ccagtcttgt gtctgcgtct 2885

gtgttgcgtg tcgtgggtgt gtgtagccaa ggtcggtaag ttgaatggcc tgccttgaag 2945

ccactgaagc tgggattcct ccccattaga gtcagccttc cccctcccag ggccagggcc 3005

ctgcagaggg gaaaccagtg tagccttgcc cggattctgg gaggaagcag gttgaggggc 3065

tcctggaaag gctcagtctc aggagcatgg ggataaagga gaaggcatga aattgtctag 3125

cagagcaggg gcagggtgat aaattgttga taaattccac tggacttgag cttggcagct 3185

gaactattgg agggtgggag agcccagcca ttaccatgga gacaagaagg gttttccacc 3245

ctggaatcaa gatgtcagac tggctggctg cagtgacgtg cacctgtact caggaggctg 3305

aggggaggat cactggagcc caggagtttg aggctgcagc gagctatgat cgcgccacta 3365

cactccagcc tgagcaacag agtgagaccc tgtctcttaa agaaaaaaaa agtcagactg 3425

ctgggactgg ccaggtttct gcccacattg gacccacatg aggacatgat ggagcgcacc 3485

tgccccctgg tggacagtcc tgggagaacc tcaggcttcc ttggcatcac agggcagagc 3545

cgggaagcga tgaatttgga gactctgtgg ggccttggtt cccttgtgtg tgtgtgttga 3605

tcccaagaca atgaaagttt gcactgtatg ctggacggca ttcctgctta tcaataaacc 3665

tgtttgtttt aaaaaaaa 3683

<210> 54
<211> 461
<212> PRT
<213> Homo sapiens

<400> 54

## Description

The present invention relates to a method for detecting non-responders to anti-TNF therapy, the use of a novel polymorphism in exon 2 and a known polymorphism in exon 6 in the gene coding for the TNF Receptor II in anti-TNF therapy, to the genes containing the polymorphism in exon 2 or exons 2 and 6, and to the peptides encoded by the respective genes.

Crohn's disease is a chronic inflammatory disorder of the intestine. It shares many clinical and pathophysiological characteristics with other autoimmune disorders including rheumatoid arthritis. A polygenic aetiology of Crohn's disease is strongly suspected (Hugo, J.P. et al. Mapping of a susceptibility locus for Crohn's disease on chromosome 16. *Nature* 379, 821-823 (1996); Cho, J. H. *et al.* Identification of novel susceptibility loci for inflammatory bowel disease on chromosome 1p. 3q. and 4q: Evidence for epistasis between 1p and IBD1. PNAS 95, 7502-7505 (1998); Satsangi J. *et al.* Two stage genome-wide search in inflammatory bowel disease provides evidence for susceptibility loci on chromosome 3, 7 and 12. Nature Genet. 14, 188-202 (1996); Hampe J. *et al.* A genomewide analysis provides evidence for novel linkages in inflammatory bowel disease in a large European cohort, Am J Hum Genet 64, 808-816 (1999)); Hampe J. *et al.* Linkage of Inflammatory bowel disease to human chromosome 6p. Am J Hum Genet 65, 1647-1655 (1999), although disease genes have not been identified yet.

Glucocorticoids are an effective short term treatment of acute relapse in most patients. However, long term maintenance of remission is difficult in many patients. It is estimated that at least 50 % of patients develop a steroid refractory and dependent disease (Munkholm P., Langholz E., Davisen M, Binder V. Frequency of glucocorticoid resistance and dependency in Crohn's disease. Gut 35, 360-362). An increased production of pro inflammatory cytokines including TNF-α (tumor necrosis factor α) in the intestinal mucosa is pivotal for the development of inflammatory relapses ( Schreiber S. *et al.* Tumor necrosis factor alpha and interleukin 1 beta in relapse of Crohn's disease. Lancet 353, 459-461 (1999)) as well as chronic inflammatory activity.

The introduction of biological agents targeting TNF-α has led to impressive clinical results in therapy of refractory Crohn's disease.

Infliximab is a monoclonal antibody against TNF-α which was recently approved for therapeutic use in refractory and/or fistulating Crohn's disease in both the United States and Europe. Further, CDP571 and D2E7 are monoclonal antibodies directed against TNF-α with different biological properties, which have either been engineered from murine antibody genes or were generated by the phage-display system, respectively. In addition, recombinant TNF-receptor based proteins have been developed (e.g. ethanercept). All bind specifically to human TNF-α (not TNF-β) but vary in their murine parts as well as the human subclass used. It is unclear whether other mechanisms in addition to neutralization of TNF-α contribute to the therapeutic effect. It appears likely that at least infliximab can bind receptor attached or membrane expressed TNF-α and leads to deletion of activated immune cells either by complement activation or induction of apoptosis (Scallon B.J., Moore M.A., Trinh H., Knight D.M., Ghrayeb J., Chimeric anti-TNF-alpha monoclonal antibody cA2 binds recombinant transmembrane TNF-alpha and activates immune effector functions. Cytokine 7, 251-259 (1995)).

TNF Receptor I (CD120a) (Smith C.A., Farrah T., Goodwin R.G., Cell 76, 959-962 (1994); Baker S.J., Reddy E.P., Oncogene 12, 1-9 (1996)) is a 55/60 kDa (455 aa residues) transmembrane glycoprotein expressed in all nucleated mammalian cells. TNF Receptor II (CD120b) is a 75/80 kDa (461 aa residues) transmembrane glycoprotein expressed primarily by cells of the hematopoietic lineage and signals thymocyte and peripheral T-cells proliferation, natural killer cell and neutrophil activation. TNF Receptor II function is not completely known. Interaction between TNF-α and TNF Receptor II leads to a slow oligomerization of receptor molecules and ligand dissociation seems to occur before receptor-signalling complex formation. Membrane bound TNF-α seems to represent the effective ligand of TNF Receptor II. The mature human TNF Receptor II is a N and O glycosylated transmembrane protein.

The gene coding for the TNF Receptor II (SEQ ID NO: 49) is located on chromosome 1p36 and consists of 10 exons and 9 introns (Santee S.M. and Owen-Schaub L.B. Human Tumor Necrosis Factor Receptor p75/80 (CD120b) gene structure and promoter characterization. Journal Biological Chemistry 271;21151-21159 (1996)). Comparison of TNF Receptor II sequences obtained by different groups has identified six potential single nucleotide polymorphisms (SNPs) in exon 4, exon 6, exon 9 and exon 10 (Pantelidis P., Lympany P.A., Foley P.J., Fanning G.C. Welsh K.I. du Bois R.M. Polymorphic analysis of the high-affinity tumor necrosis factor receptor 2. Tissue Antigens 64: 585-591). In exon 4 nucleotide substitutions at position 511-512 (all nucleotide positions refer to cDNA to mRNA sequence of GeneBank accession number M32315) give rise to an arginine to proline substitution at aa position 143; in exon 6 nucleotide substitution at position 676 corresponds to a metionine to arginine substitution at aa position 196; in exon 9 nucleotide substitution at position 1176 creates an alanine to threonine change at aa position 365; finally, the nucleotide substitution at positions 1663, 1668 and 1690 in the 3' untranslated region of exon 10. In addition to SNPs, were identified a (GATA)ₙ tetrameric repeat and a (GAA)(GGA) trimeric repeat in intron 1 (Santee S.M. and Owen-Schaub L.B. Human Tumor Necrosis Factor Receptor p75/80 (CD120b) gene structure and promoter characterization, Journal Biological Chemistry 271: 21151-21159 (1996)) and a (CA)₁₆ repeat in intron 4.

Previous studies suggest that polymorphisms in exons 6 and 10 of the gene coding for the TNF Receptor II, and amino acid exchange in TNF Receptor II potentially associated therewith, play a role in certain autoimmune diseases.

While polymorphisms in exon 4 and 9 have not been replicated, the ones in exon 6 and 10 have been studied in relation to several autoimmune diseases. Polymorphisms in exon 10 (3'UNR) at nucleotide positions 1663 and 1668 were tested in 90 patients with insulin dependent diabetes mellitus (IDDM), 101 with Graves' disease (GD) and 70 German healthy controls using Single Strand Conformation Polymorphism (SSCP) analysis (Rau H., Donner H., Usadel H. Badenhoop K. Polymorphisms of tumor necrosis factor receptor 2 are not associated with insulin-dependent diabetes mellitus or Graves' disease. Tissue antigens 49: 535-536 (1997)). Only one of the 2 polymorphic sites revealed 2 different alleles and no association was observed either with IDDM or GD in this German population. Contrasting results have been obtained for the coding mutation Met196Arg in exon 6 in relation to autoimmune diseases such as systemic Lupus erythematosus (SLE) and rheumatoid arthritis (RA). Met196Arg has been found not to be associated with RA in a Japanese population of 545 patients and 265 healthy controls (Shibue T. *et al.* Tumor necrosis factor alpha 5' flanking region, tumor necrosis factor receptor II, and HLA-DRB1 polymorphisms in Japanese patients with rheumatoid arthritis. Tissue Antigens 43(4): 753-757 (2000) Rutgeerts Gastroenterolog 1999; 117: 761-69). In another Japanese population of 81 patients and 207 normal controls Arg196Met has been found associated with SLE (Komata T., Tsuchiya N., Matsushita M., Hagiwara K., Tokunaga K. Association of tumor necrosis factor receptor 2 (TNFR2) polymorphisms with susceptibility to systemic lupus erythematosus. Tissue Antigens 53: 527-533) but not in a cohort of 128 Spanish patients and 141 controls and in 74 UK patients and 90 controls (Al-Ansari A.S., Ollier W.E.R., Villarreal J., Ordl J. Teh L.S., Hajeer A.H. Tumor necrosis factor receptor II (TNFRII) exon 6 polymorphism in systemic lupus erythematosus. Tissue Antigens 55: 97-99 (2000)).

As regards Crohn's disease, a previous study in 193 Crohn's disease patients and 93 controls had suggested that polymorphism at position -308 in the TNF promoter might be related to disease localization and steroid dependency (Luis E. et al. Tumor necrosis factor (TNF) gene polymorphism in Crohn's disease (CD): influence on disease behaviour. Clinical and Experimental Immunology 199(1): 64-68 (2000)). The microsatellite allele TNFa2 has been found associated with TNF-α and - β secretion in human mononuclear cells (Pociot *et al.* Association of tumor necrosis factor (TNF) and class II major histocompatibility complex alleles with the secretion of TNF-a and TNF-b by human mononuclear cells: a possible link to insulin-dependent diabetes mellitus. Eur J Immunol 23: 224-231 (1993)).

It has been outlined above that a new therapy against Crohn's disease involving the use of monoclonal antibodies directed against TNF-α has been developed. A one time infusion of the monoclonal antibody infliximab (commercially available as Remicade® ) at a dose of 5-20 mg/kg bodyweight results in a remission rate of approximately 30-40% without statistical differences between dose groups (Targan S.R. et al. A short-term study of chimeric monoclonal antibody cA2 to tumor necrosis factor alpha for Crohn's disease. Crohn's disease cA2 Study Group. N Engl J Med 337, 1029-1035 (1997)). Although intensely investigated, clinical parameters (e.g. disease activity, which are related to the height of mucosal TNF-α production (Reinecker *et al.* Enhanced secretion of tumor necrosis factor-alpha, IL-6 and IL-1 by isolae lamina propria mononuclear cells from patients with ulcerative colitis and Crohn's disease, Clin Exp Immunol 94, 174-181 (1993)) could not be identified as predictors for responsiveness. Non-response appears to be a stable characteristic with patients staying non-responsive even if consecutive infusions are applied. The duration of response to a single dose anti TNF-α is variable with symptoms recurring in most patients after 6-12 weeks.

Infliximab infusions are generally well tolerated, although side effects resulting from intense immunosuppression have been described (including pneumonia, reactivation of intracellular infections, sepsis and abscess formation). In addition, 5 of about 600 patients (with rheumatoid arthritis and Crohn's disease) treated in clinical studies developed a malignant lymphoproliferative disease, and one more sporadic lymphoma has been reported in the more than 30,000 patients with Crohn's disease treated in the USA.

Taking into consideration that in only 30-40% of all patients receiving infliximab an alleviation of symptoms can be observed, it is a matter of fact that 60-70% of them receive infliximab without any therapeutic advantage but with the risk of potential severe side effects. Therefore, and also in view of the high price of biological therapeutics like infliximab, a possibility to predict, whether or not a specific patient suffering from Crohn's disease will respond to the therapy, would be highly desirable.

Therefore, it is an object of the present invention to provide a simple test for detecting non-responsiveness to anti-TNF therapy, in particular infliximab therapy, in a considerable percentage of non-responders.

It is a further object of the present invention to provide a polymorphism in a gene, which polymorphism, can be used for diagnostic purposes.

It is an additional object of the present invention to provide the use of a polymorphism in a gene for anti-TNF therapy or Crohn's disease.

It is also an object of the present invention to provide a genetic sequence containing at least one polymorphism rendering the gene suitable for diagnostic purposes.

The object is achieved by a method for detecting non-responders to anti-TNF therapy, comprising testing an individual for homozygosity for at least one single nucleotide polymorphism in the gene coding for the TNF Receptor II.

The object is also achieved by a novel single nucleotide polymorphism (SNP), a transition A to G, in position 257, or 168 from the transcription starting site, in exon 2 of the gene coding for the TNF Receptor II.

The object is further achieved by the use of the single nucleotide polymorphism (SNP), the transition T to G, in position 676, or 587 from the transcription starting site, in exon 6 of the gene coding for the TNF Receptor II.

The object is, in addition, achieved by the genes having the sequences identified in SEQ ID NO 51 and SEQ ID NO 53, and by nucleotide sequences coding for the same peptides or peptides having the same immunological properties.

The SNP in exon 2 corresponds to a silent mutation at amino acid position 56 (Lys56Lys), and the SNP in exon 6 corresponds to an amino acid change at position 196 (Met196Arg). Coupling analysis reveals that both SNPs are in strong linkage disequilibrium. Therefore, the SNP in exon 2 can be used as a marker for the SNP in exon 6.

It is a great advantage of the present invention that it can be accomplished with DNA which can be derived from any cell, e.g. blood cells or other cells or body fluids, e.g. saliva, or other body parts. Preferably, DNA is derived from EDTA-blood.

It appeared likely to us that the differential response to infliximab represents a polygenic trait. Therefore, we decided to screen for mutations, by direct sequencing, the promoter, the 10 exons (including the 3'UNR) of the TNF Receptor II in 45 individuals of a study population consisting of 90 Crohn's disease patients and 180 controls, and then test the mutations present in the population, as well as known mutations in the TNF Receptor II and Receptor I, in a cohort of 90 Crohn's disease patients receiving infliximab.

3 fragments of the 5' regulatory region, the 10 exons and 1 fragment of the 3' untranslated region of the TNF Receptor II were direct sequenced in 45 individuals of the study population; the same primers, designed on the basis of the published sequence (Santee and Owen-Schaub, 1996), were used for PCR Amplification and sequencing: first fragment of the 5' regulatory region: forward primer 5'CTTCCACGAGGTGACATCTCC3' (SEQ ID NO: 1), reverse primer 5'GCCCTAATACAGGGCCAGC3' (SEQ ID NO: 2), second fragment: forward primer 5'GGACAGATTGCAGCTGGAATG3' (SEQ ID NO: 3), reverse primer 5'TAGAGCCAGACCACCTGGGT3' (SEQ ID NO: 4); third fragment: forward primer 5'AGCCTGGACAACATGGCGA3' (SEQ ID NO: 5), reverse primer 5'CCCTCGACTGAAAGCGAAAG3' (SEQ ID NO: 6); exon 1: forward primer (promoter) 5'GAGGCGTGTCCAAGGCC3' (SEQ ID NO: 7), reverse primer (intron 1) 5'GCGCGGAGTCACCACCT3' (SEQ ID NO: 8); exon 2: forward primer (intron 1) 5'ATCACCCATGGCAGAACCC3' (SEQ ID NO: 9), reverse primer (intron 2) 5'TGCCCTCACCCGGC3' (SEQ ID NO: 10); exon 3: forward primer (intron 2) 5'GACTCTGGCCTTGTTTCCTCA3' (SEQ ID NO: 11), reverse primer (intron 3) 5'GGGAAGTTGGAGGCAGGG3' (SEQ ID NO: 12); exon 4: forward primer (intron 3) 5'TGACCGTTTGCGCCCTC3' (SEQ ID NO: 13), reverse primer (intron 4) 5'GTCCCCAAGGACCTGAGCC3' (SEQ ID NO: 14); exons 5 and 6: forward primer (intron 4) 5'AGACAGAGCTCCTTGGGC3' (SEQ ID NO: 15), reverse primer (intron 6) 5'GCAGACAGAAGCAGTGAATGA3' (SEQ ID NO: 16); exons 7 and 8: forward primer (intron 6) 5'TCCTGGCTTGCTGGCTG3' (SEQ ID NO: 17), reverse primer (intron 8) 5'GAGGGCAGTGGAGACAC3' (SEQ ID NO: 18); exon 9: forward primer (intron 8) 5'GCTGACTGCTCTCCCCT3' (SEQ ID NO: 19), reverse primer (intron 9) 5'TGGGAAGAAGCAGGTGTG3' (SEQ ID NO: 20); exon 10: forward primer (intron 9) 5'GAATCTGCATCTTGGGCAGG3' (SEQ ID NO: 21), reverse primer (3' untranslated) 5'GAGGCTGCGGCTGTGGA3' (SEQ ID NO: 22); 3' untranslated region: forward primer 5'CGGTGTGGGCTGTGTCGTA3' (SEQ ID NO: 23) and reverse primer 5'CCTACAGGGCTGCCACCTC3' (SEQ ID NO: 24). Direct sequencing was conducted using BigDye Terminator (PE Biosystems) and run on an automated sequencer ABI 310 (PE Biosystems).

Direct sequencing of the 3 regions of the promoter and of the 10 exons of the TNF Receptor II confirmed the polymorphisms at amino acid position 196 (exon 6), at nucleotide position 1663, 1668 and 1690 in the 3' UNR while the amino acid position 143 in exon 4 and the nucleotide positions -1413 and -1120 in the promoter did not appear polymorphic in the 45 individuals tested. The same applies to the polymorphism in exon 9. In addition, we identified a novel polymorphism, a transition (A to G) in the third codon position of amino acid 56 (Lysine) in exon 2 (nucleotide position 168 from the transcription starting site). This mutation appears to be in strong linkage disequilibrium with the Met196 Arg in exon 6. On the basis of the data available in the literature and the results of our own sequencing we therefore decided to test in the cohort of patients receiving infliximab the following mutations: TNF Receptor I at promoter position -609 and in exon 1 at nucleotide position 36, corresponding to amino acid 12 (silent mutation); and TNF Receptor II in exon 2, silent mutation at amino acid position 56, further in exon 6, amino acid change at position 196 and in the 3' untranslated region in exon 10 at nucleotide position 1663 and 1690.

An open label, prospective multicenter clinical trial, which was conducted in 31 German centers, was specifically set up for the evaluation of pharmacogenomics and biological markers of response. Inclusion criteria (steroid/azathioprine refractory Crohn's disease, stable co-medication before and throughout the study) were similar to those used in previously published studies which established the clinical efficacy of infliximab in Crohn's disease (Present D.H. *et al*/Infliximab for the Treatment of Fistulas in Patients with Crohn's Disease N. Eng J Med 340(18): 1398-405 (1999); Targan S.R. *et al.* A short-term study of chimeric monoclonal antibody cA2 to tumor necrosis factor alpha for Crohn's disease. Crohn's disease cA2 Study Group. N Engl J Med 337, 1029-1035 (1997). The trial was conducted and monitored according to the standards of "Good Clinical Practice" (GCP). The protocol and the genetic test procedures received prior approval by all local ethics committees/institutional review boards. 96 patients with moderate to severe steroids refractory or steroid dependent Crohn's disease (10 mg or more/day) active for at least six months, with CDAI between 220 and 450, decided to participate in the treatment protocol and to provide EDTA (ethylene diamine tetraacetic acid) blood for DNA based analysis after written informed consent. At the time of the analysis clinical data was missing for 6 patients leaving the study cohort to 90 patients (55 women and 33 men and 2 unknown). The overall remission rate (as indicated by a Crohn's disease activity index below 150 points) (38 % at 4 weeks) was in the range as expected by previous studies (Targan S.R. *et al.* A short-term study of chimeric monoclonal antibody cA2 to tumor necrosis factor alpha for Crohn's disease. Crohn's disease cA2 Study Group. N Engl J Med 337, 1029-1035 (1997); Schraub L.B. Human tumor necrosis factor receptor p75/80 (CD120b) gene structure and promoter characterisation. J. Biol. Chemistry 271, 21151-21159 (1996)). On enrolment in the trial EDTA blood was obtained from each patient as well as from 180 German blood donors as normal controls. DNA was extracted by standard techniques (e.g. using DNAzol based on a guanidine-detergent lysing solution) and dispensed on 96 well plates (20 ng/well).

6 SNPs were genotyped using TaqMan (ABI 7700 PE Biosystems, Foster City, CA) allelic discrimination: 2 SNPs in the TNF Receptor I gene (12p13), one in the promoter at position -609 from the transcription starting site and one in exon 1, a silent mutation at amino acid position 12, Pro12Pro (CCA-CCG) and 4 SNPs in the TNF Receptor II gene (1p36), a silent mutation in exon 2 at amino acid position 56, Lys56Lys (AAA-AAG), a second codon position in exon 6 changing amino acid 196, Met196Arg (ATG-AGG), and 2 mutations in exon 10 in the 3' untranslated region at nucleotide position 1663 and 2007. Genotypes were assigned without knowledge of treatment response. Primers and probes (see table 1) were designed using Primer Express (PE Biosystems) and purchased from Eurogentec. PCR amplification was conducted with the termocycler 9700 (PE Biosystems) in a final volume of 10µl. The amplification conditions involved two pre-PCR steps of 2 min at 50°C and 10 min at 95°C followed by a variable number of cycles including a denaturation step at 95°C for 15 sec and an annealing step of 1 min at different temperatures for the different assays (see table 2).

While TaqMan allelic discrimination is an approved technique, the present invention should not be regarded as being limited thereto. The genotype may as well be determined by direct sequencing, RFLP (restriction fragment length polymorphism), PCR (polymerase chain reaction) - based techniques or any other technique or combination of techniques known to those skilled in the art for identifying a specific mutation.

A further particularly suitable procedure is PCR followed by restriction digestion with NlaIII ↓5'CATG↑3' (commercially available from New England Biolabs with catalogue number #125S or #125L. This enzyme cuts at position 277, 677, 941 etc. Regarding the exon 6-polymorphism, primers between 278 and 940 will yield one cut in the wild type and no cut in the mutant.

PCR-SSCP with 3' mismatches in forward and reverse primers was described in Pentelidis et al., Tissue antigens 54: 585-591 (1999) for the mutation in exon 6 as well as for exons 4, 9 and 10.

The genotypes obtained by TaqMan were checked by direct sequencing in 45 individuals of the study population obtaining in every case identical result.

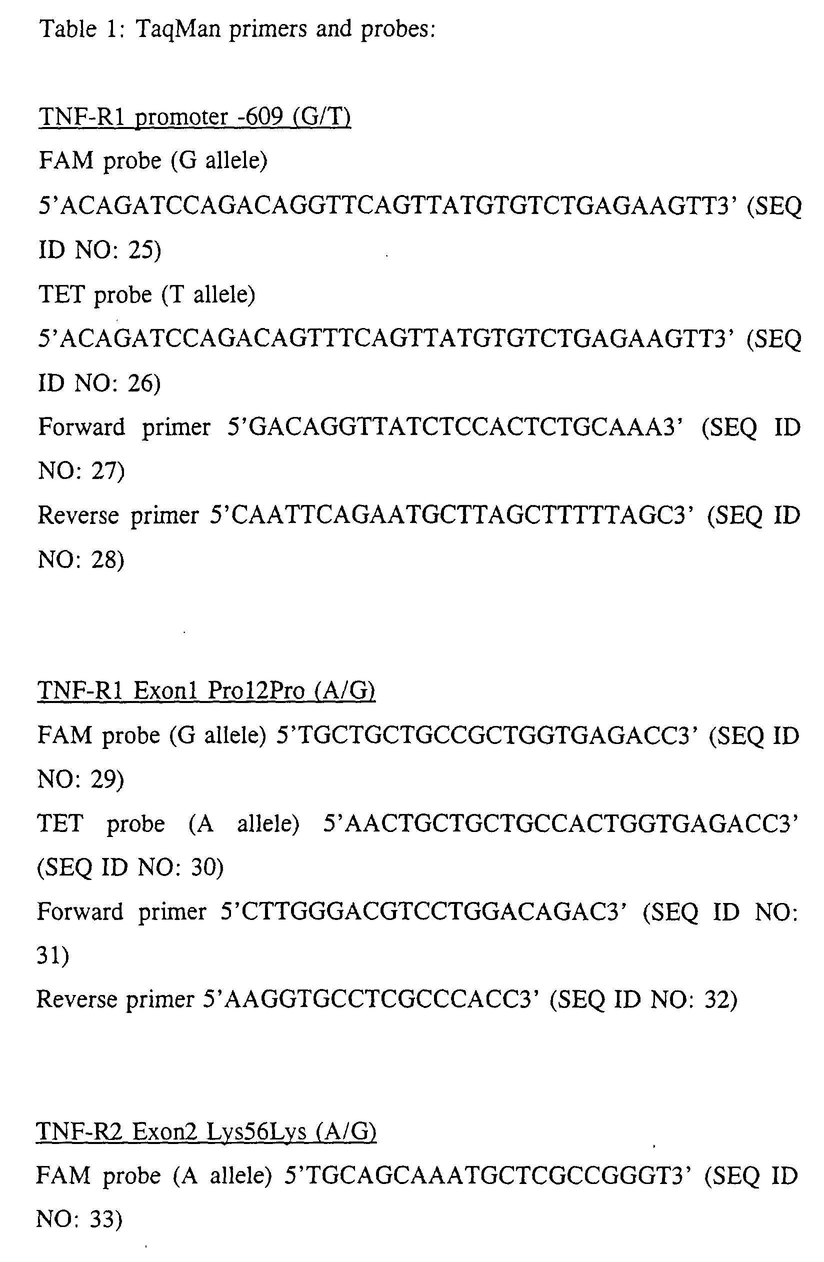

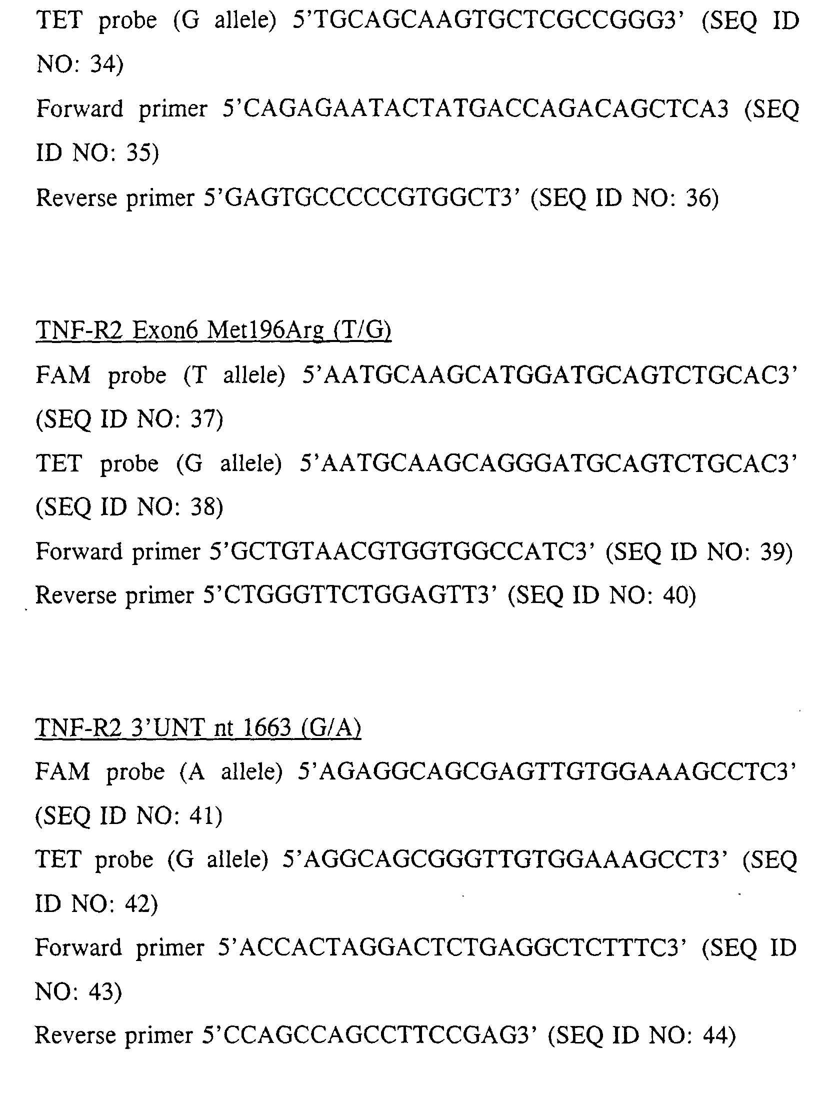

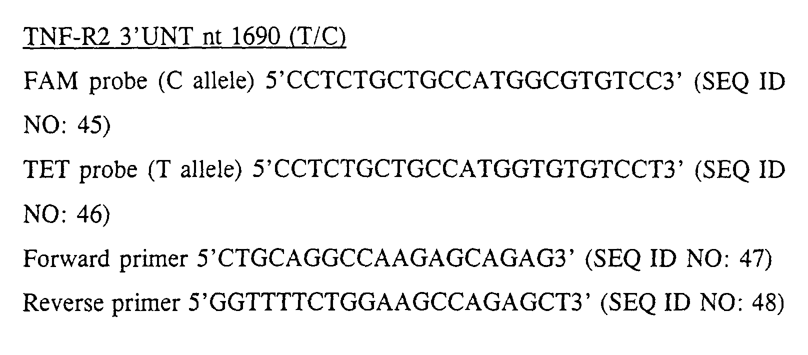

**Table 2:**

| Probes and primers concentrations and amplification conditions for TaqMan assays (ABI 7700). | | | | | | |
|---|---|---|---|---|---|---|
| SNP position | FAM Probe | TET Probe | Forward Primer | Reverse Primer | Annealing temperature and time | N. of cycles |
| TNF-R1 Promoter-609 | 200 nM | 200 nM | 300 nM | 300 nM | 1 min 64°C | 55 |
| TNF-R1 Exon 1 Pro12Pro | 200 nM | 200 nM | 300 nM | 300 nM | 1 min 60°C | 50 |
| TNF-R2 Exon 2 Lys56Lys | 200 nM | 200 nM | 900 nM | 900 nM | 1 min 60°C | 40 |
| TNF-R2 Exon 6Met196Arg | 100 nM | 100 nM | 50 nM | 900 nM | 1 min 62°C | 50 |
| TNF-R2 3'UNT nt 1663 | 200 nM | 200 nM | 300 nM | 300 nM | 1 min 60°C | 50 |
| TNF-R2 3'UNT nt 1690 | 200 nM | 200 nM | 50 nM | 900 nM | 1 min 60°C | 50 |

Of the 6 mutations tested only the one in exon 6 of the TNF Receptor II leads to an amino acid exchange (Met→ Arg at amino acid position 196). Exon 6 codes a small portion of the transmembrane region and part of the extracellular domain including the proteolytic cleavage site that produces the soluble form of TNF Receptor II. Amino acid 196 is located within the extracellular region near one of the two N-glycosylation sites. It appears to be positioned at the border of the area for which the receptor structure can be predicted and it may have a possible influence on receptor conformation (Zimmer, Lengauer, personal communication). Presently, it is not certain whether this mutation has a functional significance.

The allele and genotype frequency in the controls were comparable to the ones obtained by Ansari in Spanish and UK populations outlined above. Within our patient population, for the mutation in exon 6 there were 61 (67.8%) homozygote wild type, 23 (25.5 %) heterozygote and 6 (6.7 %) homozygote mutant. For the mutation in exon 2 there were 58 (64.4 %) homozygote wild type, 25 (27.8 %) heterozygote and 7 (7.8 %) homozygote mutant (table 3).

**Table 3:**

| Genotype and allele frequency of the Met196Arg among the 90 infliximab treated patients and 180 controls (from sex matched healthy blood donors recruited from the German population). | | |
|---|---|---|
| | Patients (total 90) | Controls (total 180) |
| **Genotype frequency** | | |
| Met196 (wild type) | 0.678 (61) | 0.672 (121) |
| Met196Arg (heterozygote) | 0.255 (23) | 0.300 (54) |
| Arg196 (mutant) | 0.067 (6) | 0.028 (5) |

| **Allele frequency** | | |
|---|---|---|
| Met196 (wild type) | 0.805 | 0.821 |
| Arg106 (mutant) | 0.195 | 0.179 |

TaqMan results were checked by direct sequencing in 45 individuals.

It was found that homozygocity for the single nucleotide polymorphism in exon 6 is always associated with non-response to infliximab (i.e. neither reaching clinical improvement (drop of the CDAI by at least 70 points) nor remission (CDAI < 150 points) resulting in a test specificity of 100 % in these individuals (table 4). Homozygote individuals show a marked reduction in clinical improvement (as indicated by a significantly smaller drop in the Crohn's disease activity index) after treatment with infliximab whereas a heterozygous genotype was not associated with an altered clinical response (table 4), i.e. the observed differences between homozygote and heterozygote individuals were at a statistically not significant level. The single nucleotide polymorphism at amino acid 196 (exon 6) of TNF Receptor II leads to a non-conservative amino acid substitution between Met(ATG) and Arg(AGG). About 10 % of non-responders are characterized by this genetic variation.

**Table 4:**

| Distribution of exon 6 genotype frequency among responders and not responders after 4 weeks from the infliximab infusion. The response has been evaluated as reduction of CDAI of 70 points, 100 points and as clinical remission (CDAI less than 150 points) | | | | | | |
|---|---|---|---|---|---|---|
| | Response 70 points (after 4 weeks) | | Response 100 points (after 4 weeks) | | Clinical remission (after 4 weeks) | |
| | **Yes** | **No** | **Yes** | **No** | **Yes** | **No** |
| Met196 (wild type) 61 patients | 36 | 25 | 32 | 29 | 18 | 42 1 not known |
| Heterozygote 23 patients | 17 | 6 | 13 | 10 | 11 | 12 |
| Arg 196 (mutant) 6 patients | 0 | 6 | 0 | 6 | 0 | 6 |

The Crohn's disease activity index incorporates 8 variables related to the disease activity: the number of liquid or very soft stools, the severity of abdominal pain or cramping, general well-being, the presence of extraintestinal manifestations, abdominal mass, use of antidiarrheal drugs, haematocrit and body weight. These items yield a composite score ranging from 0 to approximately 650. Higher scores indicate greater disease activity. Scores below 150 are compatible with remission, whereas scores above 550 indicate severe illness.

By testing patients suffering from Crohn's disease before treatment with infliximab for the mutation in exon 6, at least 10 % of those which will not respond can be detected in advance and be excluded from the useless therapy.

A second mutation in the same gene, the silent mutation in exon 2, is in a high degree of linkage disequilibrium, i.e. in almost complete linkage disequilibrium (4 discordant genotypes out of 90, i.e. there was 1 genotype heterozygote for the mutation in exon 6 and homozygote mutant for the mutation in exon 2, and 3 genotypes homozygote wild type for the mutation in exon 6 and heterozygote for the mutation in exon 2.) with the polymorphism in exon 6. Again, homozygotes are completely non-responsive to anti-TNF treatment with infliximab.

Therefore, the mutation in exon 2 can be used as a marker, to detect the same non-responders as the test for the exon 2 polymorphism.

In the TNF Receptor I gene no mutations were detected with an influence on the amino acid sequence (table 5). None of the mutations was in linkage disequilibrium with the mutation in exon 6 of the TNF Receptor II and no association with a therapeutic response was seen.

**Table 5:**

| Mutations tested and gene localisation | |
|---|---|
| **Gene and chromosomal localisation** | **Localisation and characteristics of the mutations:** |
| TNF Receptor I (p55) chromosome 12p13*** ** | Promoter -609 Pro12Pro (Pro CCA-CCG) (nucleotide position +36 MspA1, extracellular domain) |
| | |
| TNF Receptor II (p75) chromosome 1p36 | Lys56Lys (exon 2) Met196Arg (exon 6, extracellular domain) 2 mutations in the 3'UNT region |

| | |
|---|---|
| ** (Weinshenker et al., Neurology 52, 1500-1503 (1999) | |
| *** Fuchs Peter, Strehl Sabine, Dworzak Michael, Himmler Adolf and Ambors Peter Structure of the Human TNF Receptor 1 (p60) Gene (TNFR1) and Localisation to Chromosome 12p13. Genomics (1992) 13:219-224 | |

The effect of the polymorphism at position 168 in exon 2 has been shown exemplarily for Crohn's disease. The applicability of this polymorphism, however, is not restricted to Crohn's disease, but extends to any disease wherein TNF α plays a role, in particular inflammatory or malignant diseases.

## Claims

1. A method for detecting non-responders to anti-TNF therapy, comprising testing an individual for homozygosity for at least one single nucleotide polymorphism in the gene coding for the TNF Receptor II.

2. The method of claim 1, wherein anti-TNF therapy is infliximab therapy.

3. The method of claim 1 or 2, wherein anti-TNF therapy is therapy of Crohn's disease.

4. The method of any one of claims 1 to 3, wherein the at least one single nucleotide polymorphism is nucleotide substitution T/G at position 587 from the transcription starting site in exon 6 of the gene coding for the TNF Receptor II.

5. The method of any one of claims 1 to 4, wherein the at least one single nucleotide polymorphism is nucleotide substitution A/G at position 168 from the transcription starting site in exon 2 of the gene coding for the TNF Receptor II.

6. The method of claim 4 or 5, comprising identifying the mutation A/G at position 168 and/or the mutation T/G at position 587 by a technique suitable therefor.

7. The method of any one of claims 1 to 7, comprising the use of blood cells for providing DNA.

8. Use of a polymorphism at position 168 (A/G) in exon 2 of the gene coding for the TNF Receptor II for diagnostic purposes.

9. The use of claim 8 in an inflammatory or malignant or other chronic disease.

10. The use of claim 9 in Crohn's disease.

11. The use of any one of claims 8 to 10 in anti-TNF therapy.

12. Use of a polymorphism at position 587 (T/G) in exon 6 of the gene coding for the TNF Receptor II in Crohn's disease.

13. Use of a polymorphism at position 587 (T/G) in exon 6 of the gene coding for the TNF Receptor II in anti-TNF therapy.

14. A kit comprising reagents tailored to identify the polymorphism at position 168 (A/G) in exon 2 and/or the polymorphism at position 587 (T/G) in exon 6 of the gene coding for the TNF-Receptor II.

15. Gene having the nucleotide sequence identified in SEQ ID NO 51 or a nucleotide sequence coding for the same peptide or a peptide having the same immunological properties.

16. Gene having the nucleotide sequence identified in SEQ ID NO 53 or a nucleotide sequence coding for the same peptide or a peptide having the same immunological properties.

17. Peptide having the sequence identified in SEQ ID NO 52 or a peptide having the same immunological properties.

18. Peptide having the sequence identified in SEQ ID NO 54 or a peptide having the same immunological properties.
